Europäisches Patentamt

European Patent Office

Office européen des brevets

(11) Publication number : **0 481 785 A2**

# (12) EUROPEAN PATENT APPLICATION

(21) Application number : **91309587.3**

(22) Date of filing : **17.10.91**

(51) Int. Cl.$^5$ : **C12N 1/20**, C12P 19/06, C12N 9/38, // (C12N1/20, C12R1:64), (C12P19/06, C12R1:64)

(30) Priority : **18.10.90 US 599489**

(43) Date of publication of application :
**22.04.92 Bulletin 92/17**

(84) Designated Contracting States :
**DE ES FR GB IT NL**

(71) Applicant : **INTEVEP, S.A.**
**Oficina De Enlace Edificio Sucre Piso 2**
**Avenida Francisco de Miranda**
**Caracas 1070A (VE)**
(71) Applicant : **I.V.I.C. INSTITUTO VENEZOLANO**
**DE INVESTIGACION CIENTIFICA CENTRO DE**
**MICROBIOLOGICA**
**Apdo. 21827**
**Caracas 1020A (VE)**

(72) Inventor : **San Blas, Felipe, Inst.Venezolano de Inv.**
**Centro de Microbiologia, Apdo.21827**
**Caracas 1020A (VE)**
Inventor : **Moreno, Belisario, Inst.Venezolano de Inv.**
**Centro de Microbiologia, Apdo.21827**
**Caracas 1020A (VE)**
Inventor : **Antunez, Simon, Inst.Venezolano de Inv.**
**Centro de Microbiologia, Apdo.21827**
**Caracas 1020A (VE)**

(74) Representative : **Fisher, Adrian John**
**CARPMAELS & RANSFORD 43 Bloomsbury Square**
**London WC1A 2RA (GB)**

(54) **Xanthomonas campestris mutant that produces xanthan gum upon cultivation on lactose or unhydrolyzed whey.**

(57)   A strain of *Xanthomonas campestris*, ATCC No. 55,096, having the capability to assimilate lactose, is disclosed. This particular strain produces relatively large quantities of xanthan gum as well as β-galactosidase.

EP 0 481 785 A2

Jouve, 18, rue Saint-Denis, 75001 PARIS

Technical Field

The present invention relates to a new strain of the organism *Xanthomonas campestris*. This new strain produces β-galactosidase and xanthan gum in relatively high quantities when cultivated in a lactose-containing medium such as whey.

Background of the Invention

The bacterium *X. campestris* is a phytopathogenic gram-negative bacterium. When it ferments a medium containing glucose, *X. campestris* bacteria produce a water-soluble extracellular polysaccharide product known as xanthan gum, a gum that has traditionally been utilized as a viscosity-building agent or thickener in the manufacture of foodstuffs.

Xanthan gum, however, is not readily produced when *X. campestris* is maintained in a lactose-based medium such as whey. Although some species of the genus Xanthomonas, including *X. campestris,* synthesize β-galactosidase, the enzyme that is produced routinely has a low affinity for lactose.

β-galactosidase has been purified from *X. campestris* and is reported to have an affinity constant ($K_m$) for lactose of 22 millimolar (mM). Frank et al., Appl. Environ. Microbiol. 38:554-556 (1979).

*X. campestris* produces xanthan gum when grown in a medium containing glucose and hydrolyzed whey, but does not produce xanthan gum when cultivated on lactose or unhydrolyzed whey. Stauffer et al., J. Food Sci. 43:756-758 (1978). Whey is the principal by-product from the manufacture of cheese, contains about 3 to about 8 percent by weight solids, and is composed mainly of lactose, proteins, non-proteinaceous nitrogen-containing compounds, inorganic salts, and water.

Unhydrolyzed whey is a fluid medium containing a relatively low concentration of milk solids and a relatively high concentration of lactose. Typically, unhydrolyzed whey contains about 7 weight percent solids, about 12 weight percent protein and about 70 weight percent lactose.

Deproteinized and hydrolyzed whey is a fluid medium containing glucose and galactose with little whey protein or lactose present.

When lactose in deproteinized whey has been hydrolyzed by a lactase, xanthan gum is produced when *X. campestris* is cultured thereon. Charles et al., Extracellular Microbial Polysaccharides, Chapter 3, p. 27-39 (Sanford, P.A. and Laskin, A., eds., Am. Chem Soc.) (1976).

A strain of *Xanthomonas fuscans* capable of growing in a lactose-containing medium and producing an exocellular polysaccharide has been reported. Konice et al., Folia Microbiol. 22:12-18 (1977).

Strains of *X. campestris* that grow on lactose-containing whey and produce xanthan gum have been reported. Schwartz et al., Appl. Environ. Microbiol. 50:1483-1485 (1985). One such strain, BB-1, however, appears to require the presence of a yeast extract and mineral salts in the culture medium. A variant of this strain, BB-1L, has been derived that produces a functionalized whey product containing a thickening polymer when cultivated on unhydrolyzed whey but loses the ability to produce xanthan gum in the lactose-based media upon prolonged cultivation. Strain BB-1 is said to have been derived from *X. campestris* NRRL-B1459 and is also described in U.S. Patent No. 4,442,128 to Schwartz et al.

A recombinant strain of *X. campestris* that contains an *E. coli* β-galactosidase gene has also been reported. This strain produces a β-galactosidase enzyme that has over 200-fold greater specific activity than the enzyme product in the parent *X. campestris*. Walsh et al., Appl. Environ. Microbiol. 47:253-257 (1984).

The disposal of the whey waste product in dairy production is usually an expensive process. The conversion of whey to a useful food ingredient by fermentation with a stable strain of *X. campestris* that is capable of utilizing the lactose-containing whey to produce a polysaccharide such as xanthan gum provides an economical method for whey disposal that does not require the costly prior hydrolysis of the whey to produce glucose and galactose.

Summary of the Invention

The present invention is directed to a stable new bacterial strain of a *X. campestris* that grows in a lactose-based medium, such as whey, and that produces xanthan gum in relatively high quantities as well as β-galactosidase.

A particular of strain *X. campestris* is contemplated by the present invention. This strain grows in a medium containing unhydrolyzed whey and/or lactose and produces both the enzyme β-galactosidase and an extracellular polysaccharide such as xanthan gum. The production of β-galactosidase by the strain of the present invention is substantially greater than that obtainable by the parent *X. campestris*.

A biologically pure culture of the *X. campestris* strain ATCC Accession Number 55,096 is another feature of this invention. This culture grows in a medium containing unhydrolyzed whey and lactose, and produces both β-galactosidase and a functionalized whey product containing a thickening polymer, such as xanthan gum.

A method of producing an extracellular polysaccharide product such as xanthan gum is also contemplated by the present invention. In this method, a culture of *X. campestris* ATCC No. 55,096 is grown in a medium containing whey and/or lactose for a time period sufficient for the bacterial culture to produce a functionalized polysaccharide product. The polysaccharide product produced is then isolated from the culture medium in a manner known in the art.

## Brief Description of the Drawings

FIGURE 1 is a graphical representation of the utilization of lactose, whey and glucose by *X. campestris* ATCC No. 55,096 as a function of time; and

FIGURE 2 is a graphical representation of the growth of *X. campestris* ATCC No. 55,096 in various media as a function of time as compared to the growth of its parent strain in lactose as a function of time.

## Description of Preferred Embodiments

*X. campestris* ATCC No. 55,096 bacterial strain grows in a lactose-containing medium such as unhydrolyzed whey. Extracellular polysaccharides, such as xanthan gum, may also be present. These bacteria are gram negative rod, yellowish in appearance, mucoid, aerobic and mesophilic. This particular strain is a LAC$^+$ mutant and was obtained from a LAC- wild type *X. campestris* strain IVIC M2 isolated from infected leaves of tomato plant *(Lycopersicum sculentum)*.

This mutant strain, ATCC No. 55,096, is stable and has retained the LAC$^+$ property since isolation.

As used herein, the term "hydrolyzed whey" refers to whey that has been subjected to a pretreatment to convert the lactose present to glucose and galactose, such as by the action of lactase. The term "unhydrolyzed whey" refers to whey that has not been subjected to such treatment and thus contains lactose.

As used herein, the term "functionalized product," and grammatical forms thereof, refers to a polysaccharide product capable of serving as a stabilizer, thickener, emulsifier of flavor enhancer of foodstuffs, and obtainable by the action of the *X. campestris* strain of the present invention upon whey and/or an aqueous lactose solution.

The *X. campestris* strain of the present invention has been deposited in the patent culture depository of the American Type Culture Collection (ATCC), Rockville, MD 20852, U.S.A. and has been assigned ATCC Accession No. 55,096.

*X. campestris* bacteria in general produce and secrete an extracellular polysaccharide upon growth in a medium containing galactose and/or glucose. However, this bacteria typically cannot grow in a lactose-based medium, although it possesses a basal level of β-galactosidase. This enzyme in *X. campestris* has been reported to have a relatively low affinity for lactose ($K_m$=22mM). Frank et al., supra.

The present mutant strain of *X. campestris*, which is also referred to hereinbelow as *X. campestris* INTXANTHO™, on the other hand, grows in a medium containing unhydrolyzed whey and/or lactose as its carbon source. This particular strain produces β-galactosidase and an extracellular polysaccharide, such as xanthan gum, when grown in such lactose-containing medium. This particular strain can produce about 20 grams of xanthan gum per liter of culture medium.

A biologically pure culture of the *X. campestris* ATCC No. 55,096 can grow in a medium containing unhydrolyzed whey or lactose to produce both β-galactosidase and functionalized whey product containing a thickening polymer that is produced by this *X. campestris* strain. In a preferred embodiment the thickening polymer is xanthan gum.

The present LAC$^+$ mutant *X. campestris* ATCC No. 55,096 produces xanthan gum upon growth in a lactose-based medium and in a medium containing both raw (unhydrolyzed) and deproteinized whey (acid or sweet).

The LAC$^+$ mutant strain of the present invention also produces both a β-galactosidase having a relatively high affinity for lactose and xanthan gum upon growth in a suitable medium. In a preferred embodiment, the *X. campestris* ATCC No. 55,096 is grown in a lactose-based medium such as unhydrolyzed dairy whey.

In the method for producing an extracellular polysaccharide by *X. campestris* ATCC No. 55,096 a culture of this LAC$^+$ mutant strain is grown in a medium containing whey for a time period sufficient for the culture to produce a functionalized polysaccharized product in the medium. The polysaccharide product is then isolated from the medium.

The culture is usually maintained in the growth medium for about 72 to about 120 hours, and preferably for about 96 hours.

3

Derivation of *Xanthomonas campestris* INTXANTHO™

The mutant strain *X. campestris* INTXANTHO™ of the present invention was isolated from *X. campestris* strain IVIC M2 in a New Brunswick fermentator, Model C 30.

A 0.5 liter reactor was operated containing a culture medium having the composition described in Table I.

## TABLE I

### Composition of Culture Medium

| Component | Concentration (g/l) |
|---|---|
| Glucose | 20.0 |
| $(NH_4)_2HPO_4$ | 1.0 |
| $K_2HPO_4$ | 2.5 |
| Citric Acid | 2.0 |
| $MgSO_4$ | 0.1 |
| $H_3BO_3$ | 0.006 |
| $ZnCl_2$ | 0.006 |
| $FeCl_3$ | 0.00024 |
| $CaCO_3$ | 0.002 |
| HCL | 0.13 |

A semi-continuous, aerated fermentation was carried out at 30°C and at a pH of 7.0. The aeration speed was 1 liter/min. The initial stirring speed was 300 rpm which was increased during the fermentation process to 500 rpm as viscosity increased as a result of the production of xanthan gum. After 48 hours of fermentation, when xanthan gum production was detected, the fermentation vessel was emptied to half of its volume, and sterile fresh medium containing mixture of glucose (15 g/l) and lactose (5 g/l) was added in order to restore the initial volume of 500 ml. The same procedure was repeated every 48 hours, each time the glucose concentration was reduced by 5 g/l, while the lactose concentration was increased by the same proportionate amount.

Finally, glucose was completely replaced by lactose at a concentration of 20 g/l in the above-mentioned culture medium. At the end of this selection process, the broth viscosity was very high.

The raw xanthan gum yield, measured addition as the weight of the precipitate produced after addition of two volumes of 95 percent ethanol, was approximately 18 g (wet weight) per liter of broth.

If desired, a mutagenic agent such as nitrosoguanidine can be used to enhance the frequency of mutant production.

The colonies were isolated and purified by standard procedures in a petri dish containing the modified lactose medium described in Table 1, and Bacto-agar (DIFCO) in a final concentration of 15 g/l.

One defined colony with a mucoidal morphology designated INTXANTHO™, was then submitted to subsequent studies to confirm its capacity to ferment lactose and produce xanthan gum.

This isolated strain *X. campestris* INTXANTHO™ was observed to be a Gram-negative rod, yellow-pigmented, aerobic mesophyle. This strain was motile with polar (monotrichous) flagella, non-sporing, heterotrophic, and pathogenic to plants (causes necrotic infections). The isolated strain grew well on ordinary nutrient agar and produced mucoid colonies having non-diffusible yellowish pigmentation. The isolated strain liquified gelatin, did not produce detectable quantities of either hydrogen sulfide ($H_2S$) or urea, oxidized carbohydrates such as lactose with attendant production of relatively small quantities of acid but no gas. This strain was catalase and oxidase positive, but indole negative.

Production of Xanthan Gum

Example 1

The strain *X. campestris* INTXANTHO™ (ATCC No. 55,096) was tested for its capacity to grow and produce xanthan gum fermentation of a culture medium similar to that described in Table I, but in which glucose was substituted for whey. Under these conditions, the INTXANTHO™ strain was able to produce 20 g of raw xanthan gum per liter broth after 96 hours of incubation.

The results obtained proved that the strain INTXANTHO™ was able to produce similar yields of xanthan gum by fermenting either the original liquid or solid whey.

Example 2

The strain *X. campestris* INTXANTHO™ was also tested in a culture medium similar to that described in Table I, but in which glucose was substituted for lactose. Similar results were found.

Example 3

Fermentation of a whey form suspended in distilled water without addition of any of the above-mentioned ingredients of Table I, was carried out. The same concentration, 20 g/l, of xanthan gum was obtained.

In conclusion, *X. campestris* INTXANTHO™ grows well and produces large amounts of xanthan gum by fermentation of whey, rat or deproteinized, solid or liquid, suspended in distilled water without addition of any other nutrient.

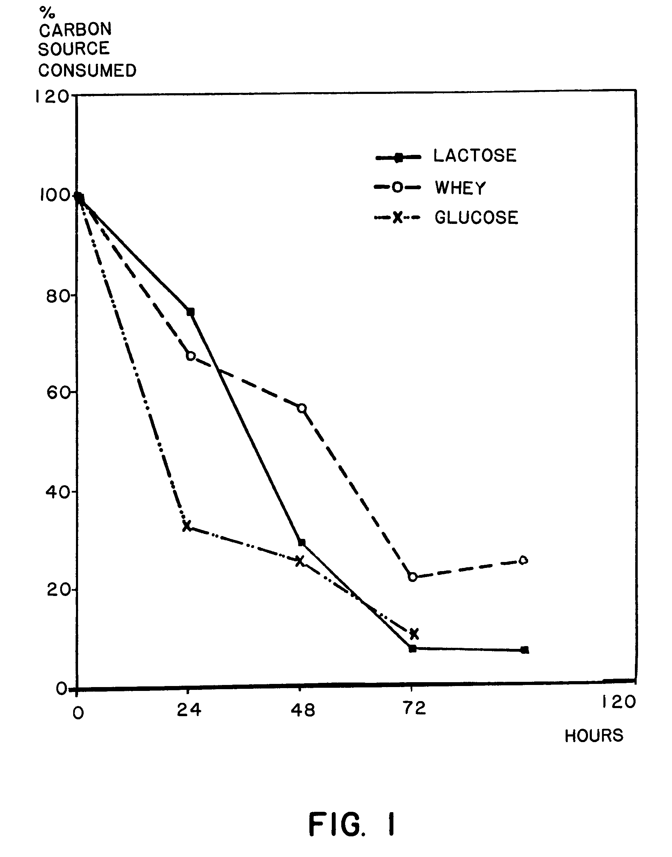

INTXANTHO™ can grow in a medium utilizing glucose, lactose or whey as the required carbon source for this process, as seen in FIGURE 1.

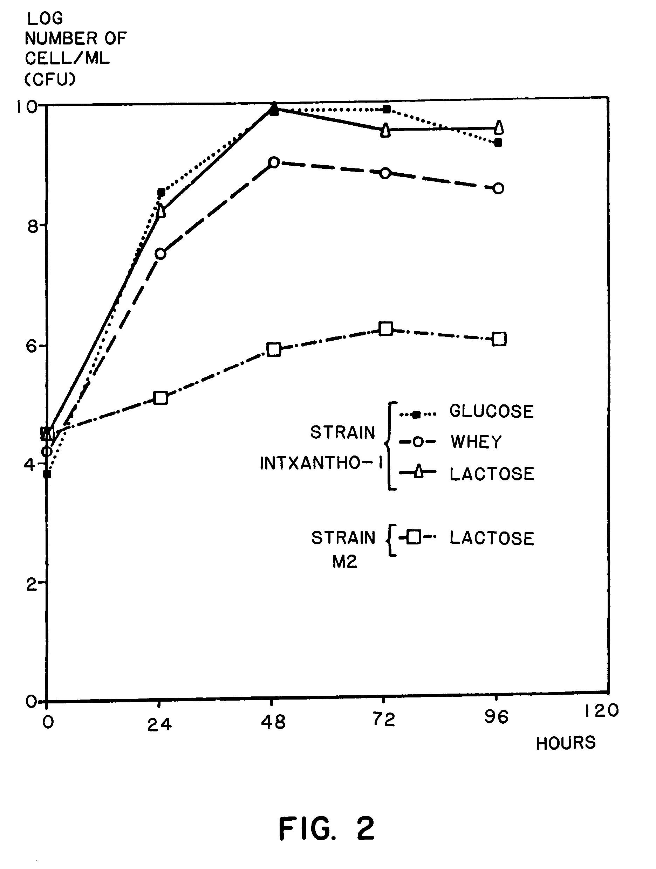

Comparative studies between the *X. campestris* strain of this invention, and the IVIC M2 strain from which it was originated, showed that the *X. campestris* mutant strain is perfectly able to ferment lactose, as whey, although the lactose is not hydrolyzed. FIGURE 2 illustrates the growth rate of the strain *X. campestris* INTXANTHO™ and compares it to the parental strain IVIC M2. The mutant strain of this invention has developed a special efficiency in used media.

Strain Characterization

*X. campestris* INTXANTHO™ (ATCC No. 55,096) was characterized with respect to *X. campestris* strains IVIC M2 and NRRL B-1459.

The following description characterizes the strain *X. campestris* INTXANTHO™.

I. Cell Morphology

a. After 18 hours of growth at 30°C in a Bacto-agar YM (DIFCO) broth, cells appear isolated or in pairs,with infrequent chain formation. The cell dimensions are less than 2.0 microns in width and 1.0 to 3.0 microns in length.

b. *X. campestris* INTXANTHO™ is heterotrophic, aerobic, and gram-negative. It is not photosynthetic, and does not form spores. It is rod-shaped and motile with polar flagella.

II. Colony Morphology

After growth for 72 hours at 30°C in Bacto-agar YM (DIFCO), the INTXANTHO™ colonies are yellow-pigmented, raised-circular and shiny. The pigment is not diffusible.

III. Biochemical Characteristics

In order to determine if the strain *X. campestris* INTXANTHO™ is physiologically different from *X. campestris* IVIC M2 and NRRL B-1459, the following tests were performed.

a. Gel, Casein and Starch Hydrolysis

Solid agar media were prepared containing, respectively, 0.5 percent casein and 0.3 percent soluble starch and were used according to the description of M.B. Gibbs and F.A. Stainer, eds., Identification Methods for Microbiologists, Academic Press, New York, N.Y. (1966), herein incorporated by reference. All strains showed a total hydrolysis of the three substrates.

### b. Hydrogen Sulfide Production

A medium of peptone-water was prepared for $H_2S$ determination, according to the procedure described in J.F. MacFaddin, Biochemical Test for Identification of Medical Bacteria, The Williams and Wilkins Company. While the NRRL B-1459 strain showed production of $H_2S$, the strains IVIC M2 and INTXANTHO™ did not produce hydrogen sulfide.

### c. Urease Production

Urea medium was prepared according to the procedure described in Biochemical Test for Identification of Medical Bacteria, referenced above. Urea production was negative for the three tested strains.

### d. Carbohydrate Assimilation Pattern

The medium described in Table I, without the carbon source, was used as the foundation carbohydrate assimilation medium. Each strain was inoculated into separate test tubes containing this medium to which one of the sugars indicated in Table II had been added. The tubes were kept at 30°C for 15 days. As observed in Table II, each strain produced a particular assimilation profile.

## TABLE II

| Carbohydrate | INTXANTHO™ | NRRL B-1459 | IVIC M2 |
|---|---|---|---|
| Glucose | + | + | + |
| Lactose | + | − | − |
| Galactose | + | + | + |
| Sucrose | + | weak | + |
| Mannitol | + | + | + |
| Xylose | + | weak | + |
| Ribose | weak | weak | weak |
| Maltose | + | + | + |

### e. Oxidase Production

The production were tested according to the procedure described in Biochemical Test for Identification of Medical Bacteria, referenced above. The three strains were positive for oxidase production.

### f. Catalase Production

The strains were tested for their capacity to produce catalase according to the procedure described in Biochemical Test for Identification of Medical Bacteria, referenced above. Each strain was highly positive for catalase production.

### g. Indole Production

The strains were tested for their capacity to produce indole according to the procedure described in Biochemical Test for Identification of Medical Bacteria, referenced above. The three strains were negative for indole production.

The foregoing description and the examples are intended as illustrative and are not to be taken as limiting. Still other variations within the spirit and scope of this invention are possible and will readily present themselves to those skilled in the art.

## Claims

1. *Xanthomonas campestris* strain, ATCC Accession No. 55,096, able to produce a recoverable amount of a polysaccharide from lactose as a carbon source.

2. The strain of claim 1, able to grow in a medium comprising unhydrolyzed whey and wherein the produced polysaccharide is xanthan gum.

3. A biologically pure culture of *Xanthomonas campestris*, ATCC Accession No. 55,096, having the ability to grow in a medium containing unhydrolyzed whey while producing a functionalized product which contains a thickening polymer.

4. The culture of claim 3, wherein said tickening polymer is xantham gum.

5. The culture of claim 3 or claim 4 wherein said culture also produces β-galactosidase.

6. A method for producing a polysaccharide product from whey comprising the steps of:
   a) culturing *Xanthomonas campestris,* ATCC Accession No. 55,096, in a medium containing whey for a time period sufficient to produce a functionalized polysaccharide product in said medium; and
   b) isolating said polysaccharide product from said medium.

7. The method of claim 6, wherein the isolated polysaccharide product is xanthan gum.

8. The method of claim 6 or claim 7, wherein said medium contains lactose.

9. The method of any one of claim 6, 7 or 8 wherein said whey is unhydrolyzed dairy whey.

10. A method for producing a mutant strain of *Xanthomonas campestris*, capable of growth in a medium containing lactose comprising:
    a) culturing a parental strain of *Xanthomonas campestris* in a nutrient medium for a time period sufficient to generate stable mutations in cells of said parental strain;
    b) transferring cells of said mutagenized parental strain to a lactose-based medium, and growing said cells therein;
    c) maintaining the transformed cells in the lactose-based medium until the viscosity of said medium increases; and
    d) isolating and purifying mutant cells of *Xanthomonas campestris* capable of growth in said lactose-based medium from said lactose-based medium.

11. The method in accordance with claim 10 wherein said lactose-based medium is unhydrolyzed dairy whey.

FIG. I

LOG
NUMBER OF
CELL/ML
(CFU)

STRAIN INTXANTHO-1
- ···■··· GLUCOSE
- -o- WHEY
- -△- LACTOSE

STRAIN M2
- -□-· LACTOSE

HOURS

FIG. 2